# EUROPEAN PATENT APPLICATION

(11) **EP 1 201 754 A1**
(43) Date of publication of application: **02.05.2002**
(21) Application number: 00944308.6
(22) Date of filing: 06.07.2000
(51) Int. Cl.: C12N 15/12, C07K 14/47, C12N 5/10, C12N 1/21, C12N 1/19, C12N 1/15, C12P 21/02, C07K 16/18, C12P 21/08, G01N 33/53, G01N 33/577

(54) **APOPTOSIS-ASSOCIATED FACTOR**

(30) Priority: 08.07.1999 JP 19417999; 18.10.1999 US 159586 P
(71) Applicant: Helix Research Institute, Kisarazu-shi, Chiba 292-0812 (JP); FUJISAWA PHARMACEUTICAL CO., LTD., Osaka-shi Osaka 541-8514 (JP)
(72) Inventor: OTA, Toshio, Fujisawa-shi, Kanagawa 251-0042 (JP); ISOGAI, Takao, Inashiki-gun, Ibaraki 300-0303 (JP); NISHIKAWA, Tetsuo, Itabashi-ku, Tokyo 173-0013 (JP); HIO, Yuri, Kisarazu-shi, Chiba 292-0812 (JP); MIYOSHI, Sousuke, Tsukuba-shi, Ibaraki 305-0031 (JP); SATOH, Susumu, Tsukuba-gun, Ibaraki 300-2436 (JP)
(74) Representative: Grünecker, Kinkeldey, Stockmair & Schwanhäusser Anwaltssozietät
(86) International application number: PCT/JP00/04516
(87) International publication number: WO 01/04300

(57) **Abstract**

A novel apoptosis-associated factor is provided. The apoptosis-associated factor of the present invention is a novel protein having DED, caspase family-cleavage domain, etc. This protein induces apoptosis in cells. Thus, a compound which is useful in regulating apoptosis can be screened by using this protein as a target. Moreover, the protein per se is useful in regulating diseases with cell proliferation.

## Description

### Technical Field

The present invention relates to an apoptosis-associated factor.

### Background Art

Apoptosis was once referred to as a programmed cell death. To survive, multicellular organisms require mechanisms to remove unwanted or unnecessary cells from living bodies thereof. Apoptosis has been understood as a pre-programmed cell death to remove cells, which have become unnecessary in the development process or alteration of generations of cells. In cells in which apoptosis has been induced, nuclear shrinkage and DNA fragmentation are observed, followed by the cell retraction with plasma membrane left intact. *In vivo*, the retracted cells are then eliminated by macrophages. In contrast to apoptosis, cell death referred to as necrosis is accompanied with the destruction of cell structure. Therefore, apoptosis whereby cells die out by themselves is often referred to as a "clean cell death".

Later, it has become known that a similar cell death as that observed in the developing process is induced by extracellular stimulus. For example, TNF and Fas are typical cytokines that induce apoptosis. Apoptosis is also induced by stimulus such as irradiation of ultraviolet ray or radiation, exhaustion of cell growth factors, or activation of certain cancer genes. Disorders such as autoimmune disorders and cancer have become understood as the results of failure to perish harmful cells due to the dysfunction of apoptosis.

By recent successive findings of various apoptosis-associated molecules, a general image of apoptosis has been gradually elucidated. However, mechanisms of apoptosis are still not elucidated.

At present, the intracellular process from response to outside stimulus to apoptosis has been described generally as follows. That is, it has been revealed that the caspase family activated by a variety of causes brings about cell death. It has been found that caspases include a number of enzymes having different substrate specificities, so that a group of these caspases has been generically called "caspase family". The caspase family is classified into three large groups based on the substrate specificity. Among them, the group 2 caspase is said to be involved in executing apoptosis. The group 3 caspase is activated in response to extracellular stimulus in upstream of the apoptosis execution cascade, functioning to activate the group 2 caspase located further downstream thereof. On the other hand, the group 1 caspase is thought to have the function to promote the production and secretion of cytokines. Caspases classified into groups 2 and 3 as well as amino acid sequences recognized by them are shown below:

| Group 2 | |
|---|---|
| Caspase-2 | DEHD |
| Caspase-3 | DEVD |
| Caspase-7 | DEVD |

| Group 3 | |
|---|---|
| Caspase-6 | VEHD |
| Caspase-8 | LETD |
| Caspase-9 | LETD |

Group 3 caspases are activated by binding of a ligand to the Fas and TNF receptors, and then, cleave group 2 caspases to activate them, resulting in transduction of signals to further downstream. Such signal transduction is referred to as "apoptosis execution cascade". In downstream of the apoptosis execution cascade, a variety of factors transmitted signals are suspected to function as the execution team to cause cell death. However, many problems remain to be solved about which factors actually cause cell death. For example, it has been demonstrated that certain caspase degrades a nuclease inhibitor ICAD (inhibitor of CAD) and then, nuclease CAD (Caspase Activated DNase) is allowed to transfer to the nuclear, followed by initiating the cleavage of chromosomal DNA characteristic of apoptosis (Enari, M. et al., Nature, 391: 43-50, 1998).

Thus, many molecules having critical functions in carrying out apoptosis are thought to be included in factors constituting the downstream of apoptosis execution cascade. These factors may become important targets in the control of apoptosis.

### Disclosure of the Invention

An object of the present invention is providing a novel apoptosis-associated factor.

In order to isolate novel human genes, the present inventors isolated many full-length cDNA clones from a human cDNA library prepared by the oligo-capping method, and then, analyzed these full-length cDNA clones for the apoptosis-associated genes based on sequence alignment. As a result, the present inventors have found that one of these full-length cDNA clones, NT2RM1000558, comprises Death Effector Domain (DED) motif and a cleavage recognition sequence for the caspase family both of which have been said to be important for the apoptotic signal transduction. This full-length cDNA clone is a gene cloned from a cDNA library of NT-2 neuron precursor cells.

NT2RM1000558 encodes a protein comprising 326 amino acid residues. Considering the amino acid sequence analysis thereof, the digestion at the cleavage recognition sequence for the caspase family is thought to remove 23 amino acid residues at the C-terminus yielding a protein comprising 303 amino acid residues. Furthermore, motifs were found in the protein of the present invention such as DED at positions 26-103, and the Nuclear Localization Signals (NLSs) at positions 100-109 as well as positions 152-174, etc.

On the other hand, a protein designated DEDD (Alexander H. Stegh et al. 1998, The EMBO Journal Vol. 17 No. 20, 5974-5986; Chandra P. Leo et al. 1998, Endocrinology Vol. 139 No, 12, 4838-4848) are known. DEDD is a protein comprising 318 amino acid residues, which has a similar structure to that of the apoptosis-associated factor of the present invention in that DEDD comprises DED, NLS, and caspase digestion domain. However, comparison of amino acid sequences of the similar DED in both proteins (DEDD: SEQ ID NO: 9) revealed only a 43% homology between them, indicating that these two proteins are clearly different proteins. Furthermore, a role of DEDD in the apoptosis execution cascade has not been fully elucidated.

Next, the present inventors confirmed activities of a protein comprising 326 amino acid residues (SEQ ID NO: 4) encoded by NT2RM1000558 and a protein resulted from its digestion comprising 303 amino acid residues (SEQ ID NO: 2). As a result, apoptosis-inducing activity in the protein comprising 326 amino acid residues was not observed, in contrast to clear apoptosis-inducing activity in the protein comprising 303 amino acid residues was observe. The present invention is achieved based on such discovery. That is to say, the present invention specifically relates to the following proteins, DNAs, and the use thereof:
[1] a polynucleotide encoding a protein having apoptosis-inducing activity, said polynucleotide selected from the group consisting of the following (a) to (d):
   (a) a polynucleotide comprising the nucleotide sequence of SEQ ID NO: 1;
   (b) a polynucleotide encoding a protein comprising the amino acid sequence of SEQ ID NO: 2;
   (c) a polynucleotide encoding a protein comprising the amino acid sequence of SEQ ID NO: 2 in which one or more amino acids are substituted, deleted, inserted, and/or added; and
   (d) a polynucleotide hybridizing under stringent conditions with a polynucleotide comprising the nucleotide sequence of SEQ ID NO: 1;
[2] the polynucleotide of [1], said polynucleotide having 60% or more homology to the nucleotide sequence of SEQ ID NO: 1;
[3] a polynucleotide encoding a precursor of a protein having apoptosis-inducing activity, said polynucleotide selected from the group consisting of the following (e) to (h):
   (e) a polynucleotide comprising a protein coding region of the nucleotide sequence of SEQ ID NO: 3;
   (f) a polynucleotide encoding a protein comprising the amino acid sequence of SEQ ID NO: 4;
   (g) a polynucleotide encoding a protein comprising the amino acid sequence of SEQ ID NO: 4 in which one or more amino acids are substituted, deleted, inserted, and/or added; and
   (h) a polynucleotide hybridizing under stringent conditions with a polynucleotide comprising the nucleotide sequence of SEQ ID NO: 3;
[4] the polynucleotide of [3], said polynucleotide having 60% or more homology to the nucleotide sequence of SEQ ID NO: 3;
[5] a polynucleotide encoding a partial peptide of a protein encoded by the polynucleotide of [1] or [3];
[6] a polynucleotide encoding molecular-evolutionarily the same gene as a gene comprising the nucleotide sequence of SEQ ID NO: 3;
[7] a polynucleotide encoding a protein that comprises the amino acid sequence of SEQ ID NO: 4 in which one or more amino acids are substituted, deleted, inserted, and/or added and that has a character dominant negative to that of a protein comprising the amino acid sequence of SEQ ID NO: 4;
[8] the polynucleotide of [7], said polynucleotide encoding the amino acid sequence of SEQ ID NO: 4 in which the amino acid sequence at position 300 to 303 has been modified to a caspase-resistant amino acid sequence;
[9] a protein encoded by the polynucleotide according to any one of [1], [3], [5], [6], and [7];
[10] a vector comprising the polynucleotide according to any one of [1], [3], [5], [6], and [7];
[11] a transformant retaining the polynucleotide according to any one of [1], [3], [5], [6], and [7], or the vector of [10];
[12] a method for producing the protein of [9], said method comprising the steps of:
   culturing the transformant of [11]; and
   recovering an expression product;
[13] a polynucleotide comprising a polynucleotide complementary to the polynucleotide according to any one of [1], [3], [5], [6], and [7] or a complementary strand thereof, said polynucleotide being at least 15 bases long;
[14] an antibody against the protein of [9];
[15] an immunological assay method comprising a step of observing an immunological reaction between the protein of [9] and the antibody of [14];
[16] a method for screening a substance regulating apoptosis, said method comprising the following steps of:
   (1) contacting, with a candidate substance, cells expressing a protein encoded by the polynucleotide of [1] or [3]; and
   (2) culturing said cells under conditions inducing apoptosis to select a candidate substance that suppresses or promotes apoptosis;
[17] a method for screening a substance regulating apoptosis, said method comprising the following steps of:
   (1) contacting, with a candidate substance, cells into which a vector comprising an expression control region for a gene comprising the nucleotide sequence of SEQ ID NO: 3 and a reporter gene operably linked downstream thereof has been introduced;
   (2) measuring activity of said reporter gene; and
   (3) selecting a candidate substance that increases or decreases the reporter gene activity in the step (2) compared with a control;
[18] use of a compound that can be obtained by the method [16] or [17], in regulation of cell proliferation or cell death; and
[19] a therapeutic agent for disorders characterized by cell proliferation or cell death, said agent comprising, as a principal ingredient, a compound that can be obtained by the method of [16] or [17].

The present invention relates to a novel polynucleotide encoding a protein having apoptosis-inducing activity. Nucleotide sequences of polynucleotides derived from human included in the polynucleotide according to the present invention are described in SEQ ID NO: 1 or 3. Amino acid sequences of proteins encoded by the polynucleotides of the present invention are also indicated in SEQ ID NO: 2 or 4. There is no particular limitation on forms of polynucleotides of the present invention as long as they can encode the protein of the present invention, which include genomic DNAs, chemically synthesized DNAs, RNAs, and so forth as well as cDNAs. Also, polynucleotides having any nucleotide sequences on which genetic codes are degenerated may be included as long as they can encode the protein of the present invention. In addition, the polynucleotide of the present invention may be fused with a polynucleotide encoding other protein and oligopeptide. Polynucleotides encoding proteins of the present invention can be isolated as described above, by the standard methods such as the hybridization method using the nucleotide sequence described in SEQ ID NO: 1 or 3, or a portion thereof as a probe, and the PCR method using a primer designed based on the information of these nucleotide sequences.

The protein of the present invention comprising the amino acid sequence described in SEQ ID NO: 2 shows apoptosis-inducing activity in mammalian cells. In contrast, the protein comprising the amino acid sequence described in SEQ ID NO: 4 does not shows apoptosis-inducing activity in mammalian cells. The amino acid sequence described in SEQ ID NO: 4 comprises, at positions 300 to 303 from the N-terminus, the amino acid sequence (DEAD) which corresponds to the Caspase Cleavage Domain. The amino acid sequence described in SEQ ID NO: 2 corresponds to that of a protein produced when the protein comprising the amino acid sequence described in SEQ ID NO: 4 is cleaved in this Caspase Cleavage Domain. Therefore, in the present invention, a protein having apoptosis-inducing activity is referred to as the "active form". On the other hand, a protein such as that comprising the amino acid sequence described in SEQ ID NO: 4, which produces the active form via a certain process but has no apoptosis-inducing activity in itself is also included in the present invention. Such protein is referred to as the "inactive form" in the present invention. Inactive form proteins in the present invention may be defined as proteins which can produce active form proteins with protease such as caspase or by the chemical treatments.

The Caspase Cleavage Domain found in the amino acid sequence described in SEQ ID NO: 4 comprises the amino acid sequence DEAD. Caspases recognizing this amino acid sequence are Caspase-3 and Caspase-7 in human. Caspase-3 and Caspase-7 belong to the group 3 caspases, constituting downstream of apoptosis execution cascade. Consequently, the active form of the protein of the present invention may be involved in important processes for the progression of apoptosis execution cascade, triggered by a variety of causes at downstream thereof. Therefore, the regulation of apoptosis may be achieved by controlling the production of the protein of the present invention or action thereof. That is to say, the present invention relates to a substance that can regulate apoptosis and a method for screening said substance. Apoptosis can be controlled with a substance that can regulate the production of the protein of the present invention or the action thereof. Such substance can be isolated by a screening method as described below.

More specifically, the inhibition of *in vivo* production of the above-described active form protein can result in the effective suppression of apoptosis progression. Alternatively, the suppression of apoptosis can be achieved by inhibiting the function of the active form protein. Expression inhibition of the above-described protein can be achieved using an antisense nucleic acid drug or a decoy nucleic acid after the transcriptional control region for the expression of the protein has been elucidated. The function of active form protein per se is effectively inhibited by altering the tertiary structure of the active site of the protein through the administration of a compound binding to the protein, or interfering with the binding of the active form protein to its target compound. Alternatively, functions of the protein of the present invention can be reduced by the dominant negative effect.

In contrast, the stimulation of the production or the activity of the above-described protein in particular cells can induce apoptosis in the cell. For example, if the production of the protein can be specifically induced in cancer cells, the cancer treatment may be safely performed. Specifically, a cancer treatment based on the present invention can be performed by inducing the expression of the gene of the apoptosis-associated factor of the present invention in cancer cells, or introducing the gene encoding the active form protein together with a promoter expressing specifically in cancer cells thereto. Alternatively, a protein useful in the cancer treatment can be also obtained by modifying the Caspase Cleavage Domain in the amino acid sequence of the inactive form protein according to the present invention with the amino acid sequence specifically recognized by a protease which is highly expressed in cancer cells. Such proteins can be used as safe drugs producing the active form protein only in cancer cells.

Furthermore, using the protein of the present invention, it is possible to obtain a novel factor associated with this protein-mediated apoptosis. For example, as in the case of the binding of the Fas-associated death domain protein (FADD) to Caspase-8, the binding of a protein having the Death Effector Domain (DED) to a factor is carried out mediated by binding between respective DEDs. Thus, a novel factor having DED, which binds to the protein of the present invention, can be obtained by utilizing DED of the protein. Specifically, by using the yeast two-hybrid system (A novel genetic system to detect protein-protein interactions. Fields, S. and Song, O. (1989) Nature 340: 245-246) with DED of the protein of the present invention as a bait, a novel factor having DED which binds to DED of the protein of the present invention can be obtained from cDNA libraries derived from mammalian cells and tissues.

Furthermore, for example, by using the inactive form protein according to the present invention, a novel caspase that recognizes the caspase cleavage domain thereof can be also obtained. Specifically, first, a labeled inactive form protein as a substrate is reacted with a protein solution containing activated caspases. Subsequently, these caspases can be isolated by detecting the conversion to the active form protein from the inactive form protein of the present invention as an indicator in the reaction solution using SDS-PAGE, etc. Since the protein of the present invention is a novel protein, a caspase which digests the protein may be a novel caspase other than Caspase-3 and Caspase-7.

Alternatively, when the active form protein of the present invention causes apoptosis in conjunction with other factors, these factors can be isolated using the active form protein of the present invention. Examples of these factors may be proteins such as FADD and Caspase-8 which serve as factors playing the critical role for inducing the function of the apoptosis signal by CD95 (Fas). Such novel proteins having DED and involving in the apoptosis signal can be isolated by measuring apoptosis in mammalian cells such as HEK293T cells, induced by coexpression of the proteins with the active form protein of the present invention.

The protein of the present invention can be prepared as a recombinant protein or a natural protein. For example, a recombinant protein can be prepared by introducing a vector containing a DNA insert encoding the protein of the invention into an appropriate host, and purifying the expressed products from the transformant, as described below. Alternatively, it is possible to prepare the protein of the invention by in vitro translation ("On the fidelity of mRNA translation in the nuclease-treated rabbit reticulocyte lysate system." Dasso M.C., and Jackson R.J. (1989) Nucleic Acids Res. 17: 3129-3144). On the other hand, a natural protein can be prepared, for example, by utilizing an affinity column which is bound with the antibody against the protein of the invention, as described below ("Current Protocols in Molecular Biology" Ausubel et al. edit. (1987) John Wily & Sons, Section 16.1-16.19). The antibody used in the preparation of an affinity column can be a monoclonal antibody or polyclonal antibody.

In addition, the present invention includes a polynucleotide encoding a protein functionally equivalent to the protein comprising the amino acid sequence described in SEQ ID NO: 2 or 4. Herein, the term "functionally equivalent" means that a subject protein is functionally equivalent to the active form protein of the present invention when the protein has an activity to induce apoptosis. Alternatively, a protein which can result in the active form protein having apoptosis-inducing activity through certain processes is functionally equivalent to the inactive form protein of the present invention. The process to produce the active form protein from the inactive form protein is not limited. For example, when a protein comprising the amino acid sequence described in SEQ ID NO: 4 is used, the digestion at the caspase cleavage domain thereof is required for the production of the active form protein. Substitution of this domain with the recognition sequences of other caspases or further proteases other than caspases may result in inactive form proteins producing active form proteins by the action of corresponding enzymes. Apoptosis-inducing activity can be confirmed, for example, by the expression of a gene encoding the protein in mammalian cells and the observation of apoptosis in the cells as described in the working examples. Apoptosis can be found by considering morphological changes of cells and chromosomal DNA fragmentation as an indicator.

One skilled in the art would be able to prepare proteins functionally equivalent to the proteins identified in the working examples below, for example, by using a method for introducing mutations into the amino acid sequences of proteins (e.g. site-directed mutagenesis, Current Protocols in Molecular Biology edit. Ausubel et al. (1987) Publish. John Wily & Sons Section 8.1-8.5). Such proteins might occur due to spontaneous mutation of amino acids in nature. The present invention also includes a protein having an amino acid sequence in which one or several amino acid residues are different from those found in a sequence of any one of the proteins identified in the working examples below (SEQ ID NO: 2 or 4) due to a substitution, deletion, insertion and/or addition, as long as the protein retains a function equivalent to the proteins identified in the working examples below.

Number or sites of amino acid mutations in the protein are not limited, as long as the protein function is retained. The number (percentage) of mutations is typically 10% or less, preferably 5% or less, and more preferably 1% or less of the total amino acids. Preferably, in view of maintaining protein function, substituting amino acids may have properties similar to the amino acids to be substituted. For example, Ala, Val, Leu, Ile, Pro, Met, Phe, and Trp are all classified into nonpolar amino acids, and they are thought to share common properties. Uncharged amino acids include Gly, Ser, Thr, Cys, Tyr, Asn, and Gln. Acidic amino acids include Asp and Glu, and basic amino acids include Lys, Arg, and His.

Alternatively, a protein functionally equivalent to the protein identified in the working examples below can be isolated using a hybridization or gene amplification technique well known to one skilled in the art. More specifically, using the hybridization technique (Current Protocols in Molecular Biology edit. Ausubel et al. (1987) Publish. John Wily & Sons Section 6.3-6.4), one skilled in the art would routinely be able to isolate a polynucleotide showing a significant homology to any one of the polynucleotide sequences encoding the proteins identified in the working examples below (SEQ ID NO: 1 or 3) by using a polynucleotide sequences, or a portion thereof, and obtain a functionally equivalent protein from the polynucleotide isolated. The present invention includes a protein encoded by a polynucleotide hybridizing to a polynucleotide encoding a protein identified in the working examples below, as long as the proteins are functionally equivalent. A functionally equivalent protein can be isolated from animals including, but not limited to, vertebrates such as humans, mice, rats, rabbits, pigs, and cattle. From these animals, one can isolate genes that originated from molecular-evolutionarily the same gene that encodes an apoptosis-associated factor of the present invention. As used herein, the term "genes that originated from molecular-evolutionarily the same gene" refers to genes that are rationally judged to have evolved from one ancestor gene from which the human gene of the present invention evolved in the course of molecular-evolution. This judgment is based on polynucleotide sequence analysis of the genes or analysis of their physiological roles and such. Such genes maintain a significant nucleotide sequence homology among them.

Stringent hybridization conditions for isolating a polynucleotide encoding a functionally equivalent protein are, typical washing conditions such as "1x SSC, 0.1% SDS, 37 °C". More stringent conditions are, for example, "0.5x SSC, 0.1% SDS, 42°C", and even more stringent conditions are, for example, "0.1x SSC, 0.1% SDS, 65°C". The more stringent the hybridization conditions become, the more homologous to the probe sequence the polynucleotide is expected to be. Note that the above combinations of SSC, SDS, and temperature are given only for illustration, and one skilled in the art can achieve the same level of stringency by combining these factors appropriately to determine the hybridization conditions. The factors include those described above, or other factors (e.g. probe concentrations, length of probes, reaction time, etc.).

In general, a protein isolated using such hybridization techniques shows a significant homology in the nucleotide sequence encoding the protein, or in its amino acid sequence, compared to the sequence of a protein of the present invention, shown in SEQ ID NO: 2or 4. "Significant homology" refers to a sequence identity of at least 60% or more, preferably 70% or more, more preferably 80% or more (e.g. 90% or more). Sequence homology can be determined using the BLAST 2 search algorithm (Altschul, S.F. et al, 1997, Gapped BLAST and PSI-BLAST: a new generation of protein database search programs, Nucleic Acids Res. 25: 3389-3402).

A gene-amplification technique (PCR) (Current protocols in Molecular Biology edit. Ausubel et al. (1987) Publish. John Wiley & Sons Section 6.1-6.4)) can be used to design primers based on a part of any of the polynucleotide sequences identified in the working examples below (SEQ ID NO: 1 or 3), and to isolate a polynucleotide fragment that shows a significant homology to the above polynucleotide sequence or a portion thereof, to obtain a protein functionally equivalent to the protein identified in the working examples.

Alternatively, the present invention relates to a DNA encoding a protein having a character dominant negative to that of the protein of the present invention. A polynucleotide encoding a protein having a dominant negative character has the function to delete or reduce the activity of endogenous wild-type protein inherent in cells by the expression thereof. For example, substitution of the region corresponding to the DEAD sequence in the apoptosis-associated factor of the present invention with an amino acid sequence which is not cleaved by caspase can result in an inactive protein which never produces the active form protein. Expression of a gene encoding such amino acid sequence in cells suppresses the expression of apoptosis-associated factor of the present invention by the dominant negative effect (competitive inhibition of the inactive form). Therefore, the progression of apoptosis can be inhibited by introducing patients having the above-described disorders with a gene encoding the inactive form protein to express using gene transfer technique with virus vectors, etc.

The present invention also provides partial peptides of the protein of the present invention. These partial peptides are useful as the immunogen to obtain antibodies for the protein of the present invention. In particular, a partial peptide comprising an amino acid sequence specific to the protein of the present invention with low homology to other proteins is expected as the immunogen to yield antibody with a high specificity to the protein of the present invention.

In addition, partial peptides of the apoptosis-associated factor of the present invention or its inactive form protein include partial peptides containing, for example, DED, which is a highly conserved amino acid sequence found among a number of apoptosis-associated factors. DED corresponds to 78 amino acid residues at positions 26 to 103 of the protein (common in both the active and inactive forms) of the present invention. Since DED has been the to be a region responsible for the signal transduction in the apoptosis execution cascade, this region is thought to play an important role also in the maintenance of the activity of the protein of the present invention. In addition, the nuclear localization signal (NLS) domain found in the protein of the present invention may also be an important portion to support the activity thereof. Therefore, a peptide containing this region is useful as a target for screening compounds that modify the activity of this protein.

A partial peptide of the present invention comprises at least 7 amino acid residues, preferably 9 or more amino acid residues, more preferably 12 or more amino acid residues, even more preferably 15 or more amino acid residues. The partial peptide of the present invention may be produced by, for example, genetic engineering, a well-known technique for peptide synthesis, or cleavage of the proteins of the present invention with an appropriate peptidase.

The present invention also provides expression vectors comprising any one of the polynucleotides mentioned above. Furthermore, the present invention relates to polypeptides mentioned above, transfectants harboring any of the expression vectors mentioned above, and a method for producing an apoptosis-associated factor or a partial peptide thereof. Such a method comprises culturing a transfectant and isolating a protein of the present invention from the culture. Moreover, the present invention provides the protein or partial peptide produced by the above method.

When producing polypeptides by means of genetic recombination, the type and extent of glycosylation of a polypeptide of interest would differ depending on the type of host cell. Furthermore, in the method of so-called "secretory production" of polypeptides, it is well known to one skilled in the art that (N- and/or C-) terminal amino acid sequences of precursor peptides expressed in host cells would undergo processing by signal peptidases and such to produce polypeptides having various terminal sequences. Therefore, one skilled in the art would easily understand that such polypeptides are also included in the proteins of the present invention.

The working example described below illustrates only an example of constructing a vector that functions in mammalian cells as an expression vector. However, since the polynucleotide sequences encoding the proteins of the present invention are disclosed herein, it would be easy for one skilled in the art to construct an expression vector that can express and produce a protein of the present invention when such a vector is introduced into a fungal host cell, such as a yeast, or a prokaryotic host cell. Therefore, the present invention includes expression vectors constructed using any methods known in the art based on the polynucleotide sequences of the present invention.

Microbial cells that can be used for the expression of the polynucleotides encoding the proteins of the present inventions include, for example, prokaryotic bacteria (e.g. Escherichia coli and Bacillus subtilis) and eukaryotic yeasts (e.g. Saccharomyces cerevisiae). Mammalian cells include cultured human cells and cultured animal cells. Moreover, cultured plant cells can be used.

Examples of microorganisms include bacteria of the genus Escherichia and baker's yeast. More specifically, the host microorganism includes, for example, the following strains:
bacteria belonging to the genus *Escherichia*
   *E. coli* HB101 (ATCC 33694),
   *E. coli* HB101-16 (FERM BP-1872),
   *E. coli* MM294 (ATCC 31446),
   *E. coli* DHl (ATCC 33849), etc.; and
Baker's yeast
   *S. cerevisiae* AH22 (ATCC 38626), etc.

Examples of mammalian cells include HEK293 cells derived from human embryonic kidney cells, mouse L929 cells, Chinese hamster ovary (CHO) cells, etc.

Generally, expression vectors are constructed with, at least, a promoter, an initiation codon, a polynucleotide encoding the amino acid sequence of any of the proteins of the present invention, a termination codon, and a self-replication unit, when prokaryotes, bacteria, particularly E. coli are used as host cells. When eukaryotic cells such as yeast and mammalian cells are used, expression vectors are preferably constructed with, at least, a promoter, an initiation codon, a polynucleotide encoding the amino acid sequence of any of the proteins of the present invention, and a termination codon. Additionally, an enhancer sequence, 5'- and 3'-untranslated regions for the proteins of the present invention, a polyadenylation site, and a self-replicationunitmaybeintegrated.

The self-replication unit preferably comprises a selectable marker for transfectants (e.g. resistance to ampicillin). In the case of expression vectors using bacteria as host cells, the term "promoter" means a promoter-operator region containing a promoter, operator, and a Shine-Dalgarno (SD) sequence (e.g. AAGG, etc.). Examples of such promoters include conventional promoter-operator regions (e.g. the lactose operon, PL-promoter, trp-promoter, etc.). An Example of a promoter for expression vectors used in yeast host cells includes the pho5 promoter. Additionally, to facilitate purification, basic amino acids having affinity for chelated metal ions can be added to either end of a protein of the present invention.

When basic amino acids are added, a primer having, at its 5'-end, a nucleotide sequence sequentially coding for desired amino acid residues can be used for PCR to introduce an oligonucleotide at either end of a gene of interest. Histidine, lysine, arginine, and such can be used as basic amino acids.

Examples of promoters used in expression vectors in mammalian cells include the HTLV-LTR promoter, early and late SV40 promoters, CMV promoters, the mouse metallothionein I (MMT) promoter, etc. A preferred example of an initiation codon is the methionine codon (ATG).

Apolynucleotide encoding an amino acid sequence of the proteins of the present invention may be obtained by, for example, partial or complete synthesis of nucleotides using a DNA synthesizer. Alternatively, it can be obtained from a human cDNA library by using a probe or primer set that is designed based on a nucleotide sequence as shown in SEQ ID NO: 1 or SEQ ID NO: 3. The genomic DNA encoding the proteins of the present invention can also be prepared by treating genomic DNA according to a conventional method (e.g. digestion with restriction enzymes, dephosphorylation by bacterial alkaline phosphatase, phosphorylation by T4 polynucleotide kinase, and ligation with T4 DNA ligase). Furthermore, the genomic DNA thus obtained can be used to demonstrate the transcriptional initiation site of a gene of the present invention located on the genome. This allows one to specify expression-regulatory regions located upstream of the gene. Regulatory regions, such as promoters and enhancers, which would control expression of a gene encoding a protein of the present invention, are useful as target regions for detecting aberrant expression of a protein of the present invention. Regulation of gene expression can be achieved using decoy nucleotide pharmaceuticals that target such regions.

Among proteins of the present invention, the active form protein has a lethal action at least to human cells. Therefore, in mammalian cells, it is desirable to express the active form protein in conjunctionwith a promoter capable of inducing the expression thereof. A temperature-sensitive promoter and drug-sensitive promoter are known as such promoters. These promoters are activated only the specified culture conditions. Therefore, the expression can be induced at the stage in which cells are fully grown up.

Essential procedures of this invention such as DNA cloning, construction of each plasmids, transfection of host cells, culturing of transformants, recovery of proteins from cultures, and so forth can be carried out according to the methods well known to those skilled in the art or described in the references (Molecular Cloning 2^{nd} edition, T. Maniatis et al., Cold Spring Harbor Laboratory (1989); DNA Cloning, D. M. Glover. IRL PRESS (1985), etc.).

The host cells of the present invention include cells used for functional analysis of the apoptosis-associated factor of the present invention and those used for screening inhibitors or enhancers of the functions of the proteins. Introduction of a vector into host cells may be conducted using any of the methods including, for example, calcium phosphate precipitation, electroporation (Current protocols in Molecular Biology edit. Ausubel et al. (1987) Publish. John Wiley & Sons. Section 9.1-9.9), the lipofectamine method (Gibco-BRL), and microinjection. Preparation of the proteins of the present invention from transfectants may be conducted using any of the isolation and purification methods well known to one skilled in the art.

The present invention also provides a polynucleotide comprising at least 15 nucleotides, which is complementary to any of the polynucleotide sequences shown in SEQ ID NO: 1 or3, or to a complementary strand thereof. As used herein, the term "complementary strand" refers to one strand of a double-stranded polynucleotide that forms base pairs of A:T (A:U) and G:C with the other strand of the polynucleotide. Also, "complementary" is defined as not only sequences that completely match a continuous nucleotide region of at least 15 nucleotides, but also sequences having a homology of at least 70%, preferably at least 80%, more preferably 90% or more, and most preferably 95% or more to that region. Sequence homology can be determined according to the algorithm as described in this description.

Such a polynucleotide can be used as a probe for isolating and detecting the DNA or RNA encoding a protein of the invention, or as a primer for amplifying a polynucleotide. When used as a primer, the DNA usually comprises 15-100 bp, and preferably, 15-35 bp. When used as a probe, the DNA comprises the entire sequence of a DNA of the invention, or at least a part of it, and comprises at least 15 bp. When used as a primer, the 3'-region of the polynucletide must be complementary, but the 5'-terminal may contain additional sequences, such as a restriction enzyme recoginition site or a tag.

The polynucleotides of the present invention can be used for testing or diagnosing aberrations in the proteins of the present invention. For example, the polynucleotides of the present invention can be used as probes or primers to test aberrations in gene expression by Northern hybridization or RT-PCR. Alternatively, the polynucleotides of the present invention as primers can be used for polymerase chain reaction (PCR) to amplify the polynucleotides encoding the proteins of the present invention or the expression regulatory region thereof by genomic DNA-PCR or RT-PCR, and thus abnormalities of the sequence can be tested or diagnosed using RFLP analysis, SSCP, and sequencing. As used herein, the term "expression" includes transcription and/or translation. Expression analysis of the polynucleotides of the present invention may allow the testing and diagnosing of gene expression at the transcriptional level. Gene expression at the translational level may be tested or diagnosed by using antibodies raised against the proteins of the present invention as described below.

Moreover, "a polynucleotide comprising at least 15 nucleotides, which is complementary to any of the polynucleotide sequences as set forth in SEQ ID NO: 1 and 3, or to a complementary strand thereof" include antisense polynucleotides for inhibiting the expression of the proteins of the present invention. Antisense polynucleotides comprise at least 15 bp or more, preferably 100 bp or more, more preferably 500 bp or more, and usually 3000 bp or less, preferably 2000 bp or less.

Such antisense polynucleotides are expected to be applied in gene therapy for diseases caused by aberrations (in function or expression) in the proteins of the present invention. Specifically, apoptosis is an important step in damaging to tissue caused by ischemic disease. Thus, if the expression of the present protein in the tissue in which a blood flow disorder occurred is inhibited, tissue damage accompanying ischemic disease can be reduced. An antisense polynucleotide can be prepared, for example, by utilizing the phosphorothioate method based on the sequence information of a polynucleotide sequence shown in SEQ ID NO: 1 or 3 ("Physicochemical properties of phosphorothioate oligodeoxynucleotides." Stein (1988) Nucleic Acids Res. 16: 3209-3221).

A polynucleotide or antisense polynucleotide of the present invention can be used in gene therapy, for example, by administrating it to a patient by utilizing the in vivo or ex vivo method using vectors such as retrovirus vectors, adenovirus vectors, and adeno-associating virus vectors, or non-virus vectors such as liposomes.

The present invention also relates to an antibody capable of binding to a protein of the invention. The form of the antibody is not especially restricted; it includes polyclonal antibodies, monoclonal antibodies, or portions thereof, which are capable of binding to an antigen. It also includes antibodies of all classes. Furthermore, specialized antibodies such as humanized antibodies are also included.

If the antibody is a polyclonal antibody, it can be obtained according to the standard method by synthesizing a protein of this invention, or a partial peptide thereof, and immunizing rabbits with the protein or peptide (Current Protocols in Molecular Biology (1987) Ausubel et al. edit, John Wily & Sons, Section 11.12-11.13). If the antibody is a monoclonal antibody, it can be obtained by immunizing mice with a protein of this invention, or a partial peptide thereof, and producing a hybridoma cell by fusing spleen cells and myeloma cells (Current Protocols in Molecular Biology (1987) Ausubel et al. edit, John Wily & Sons, Section 11.4-11.11).

An antibody capable of binding to a protein of the present invention can be used for purifying a protein of the invention, and also for detecting and/or diagnosing aberrations in the expression and the structure of the protein. Specifically, proteins may be extracted from tissues, blood, or cells, and methods such as western blotting, immunoprecipitation, or ELISA can be used for the above purpose.

Furthermore, an antibody capable of binding to the proteins of the present invention may be utilized for treating diseases associated with the protein. If the antibody is used for treating patients, a human antibody or humanized antibody is desirable in terms of their low antigenicity. Human antibodies can be prepared by immunizing a mouse in which the immune system has been replaced with that of a human ("Functional transplant of megabase human immunoglobulin loci recapitulates human antibody response in mice" Mendez M.J. et al. (1997) Nat. Genet. 15: 146-156, for a reference). Humanized antibodies can be prepared by recombination of the hyper variable region of amonoclonal antibody (Methods in Enzymology (1991) 203: 99-121).

The present invention provides a method of screening for compounds regulating the activity of the proteins of the present invention. Since the proteins of the present invention causes cell death, compounds that suppress the activity of products of the gene may be useful as therapeutic agents or reagents for suppressing cell death. This screening method comprises the following steps of:
(1) contacting a candidate compound with cells expressing the protein of the present invention; and
(2) culturing the cells under the conditions for inducing apoptosis to select a candidate compound which suppresses or promotes apoptosis.

Cells expressing the protein of the present invention include those expressing the active or inactive form protein.

Since the expression of the active form protein in cells directly causes apoptosis, these cells per se are not expected to be useful for the effective screening. Thus, an expression plasmid in which the gene (of the active form protein) is linked downstream of a controllable promoter sequence can be used. A controllable promoter can include, for example, a metallothionein promoter or the like. Animal cells such as HEK293 are transformed with a plasmid which expresses the gene encoding the active form protein of the present invention under the control of a promoter as described above. The resulting transformant cause cell death under the conditions in which the promoter has been activated.

For example, the transformant is plated on 96-well multiplates, and then a subject candidate compound is added to each well, followed by the activation of the promoter. As a result, compounds to regulate the affect of the protein of the present invention can be selected by screening candidate compounds to suppress (or promote) apoptosis compared with a control. This method can be applied to any host cells in which cell death is induced in a similar system besides mammalian cells, regardless of eukaryotes or prokaryotes.

On the other hand, when the inactive form protein is to be expressed, the production of the active form protein is induced by exposing host cells to the conditions for producing the active form protein. Specifically, if the inactive form protein is to be converted to the active form by the cleavage at the caspase recognition domain, the inactive form protein is expressed in host cells expressing the required caspase. Alternatively, for a transformant in which the caspase recognition domain is replaced with an amino acid sequence to be cleaved by a protease other than caspase, host cells producing the required protease may be used. Such protease may be artificially transferred into cells, or an enzyme originally contained in the cells can be used. In either case, since the protease activity required for the production of the active form protein directly causes apoptosis, the production thereof is desirable to be controllable.

Screening of the present invention is carried out with apoptosis as an indicator. The method for detecting apoptosis is well known in the art. Specifically, apoptosis is detected in a usual method considering changes in cell form and DNA fragmentation as an indicator. Morphological changes of cells due to apoptosis can be observed as changes in the stained image by the Giemsa stain or fluorescence stain. Alternatively, DNA fragmentation can be detected by TUNEL method (Gavriel, Y. et al., 1992, J Cell Biol. 119: 493-501). Furthermore, a method for detecting the early stage of apoptosis includes the Annexin V method (Vermes, I. et al., J. Immunol. Methods, 1995, Jul 17; 184 (1): 39-51), wherein the fluorescence label, Annexin V, bound to structurally altered cytoplasmic membranes is measured with a flow cytometer, and so forth.

Compounds selected by the screening method of the present invention include those which regulate the action of the protein of the present invention by the following systems, wherein:
(1) compounds inhibit (or promote) the process whereby the protein of the present invention cleaved so as to be converted to active form thereof;
(2) compounds inhibit (or promote) the production of the protein of the present invention per se, and
(3) compounds inhibit (or promote) the cleavage of the inactive form protein of the present invention by the activated caspase.

Increase of the gene expression product of the present invention in cells may easily promote cell death triggered by certain stimulus. Therefore, compounds that control the expression of the gene or that promote cell death may be useful as the therapeutic agents or reagents for a variety of disorders (e.g., cerebral infarction, cancer, or etc.). The control of gene expression can be observed by the reporter assay. The present invention relates to a method for screening apoptosis-regulating substances according to a reporter assay in which the expression-regulating region of the gene comprising the nucleotide sequence described in SEQ ID NO: 3 based on the present invention. A screening method of the present invention comprises the following steps of:
(1) contacting a candidate compound with a cell, wherein a vector has been introduced into said cell, said vector comprising: an expression regulatory region of a gene of SEQ ID NO: 3, and, a reporter gene operably linked downstream of the expression regulatory region;
(2) measuring the activity of the reporter gene; and,
(3) selecting the candidate compound that increases or decreases the reporter activity measured when compared to the control.

A regulatory region that controls expression of a gene comprising a nucleotide sequence of SEQ ID NO: 3may be cloned from chromosomal DNA by any method well known in the art. For example, S1 mapping is well known as a method for specifying transcription initiation sites ("Isolation of Transcriptional Regulatory Region" and "Isolation and Purification of Transcription Factors" in Department Oncology, The Institute of Medical Science (ed.), "Current Protocols for Cellular Engineering", Cell Technology, Separate Volume 8, pp. 362-374, Shujunsha Co. Ltd., 1993). In general, screening of a human genomic library using a 15-100bp, preferably 30-50 bp fragment at the 5' terminus of the gene may allow cloning of the regulatory region DNA as a cloned gene comprising the expression regulatory region. The cloned DNA thus obtained often contains 10 kb or longer sequence of the 5'-untranslated region of the gene. Then, the 5'-terminal region of the cloned DNA is shortened or fragmented by treating with, for example, an exonuclease. The minimal essential unit for maintaining the activity of the regulatory region can be found by evaluating the expression level or regulation of expression of the reporter gene using a sequence comprising a shortened expression regulatory region (deletion study).

A computer program that predicts expression-regulatory regions of genes using Neural Network is widely known (http://www.fruitfly.org/seq_tools/promoter.html, Reese, M.G., et al, "Large Scale Sequencing Specific Neural Networks for Promoter and Splice Site Recognition" Biocomputing: Proceedings of the 1996 Pacific Symposium, edited by Lawrence Hunter and Terri E. Klein, World Scientific Publishing Co, Singapore, January 2-7, 1996). Alternatively, the minimal essential unit for maintaining the activity is predicted using a program such as the Promoter Scan program that searches for a transcription factor binding sequence and predicts the expression regulatory region (http://biosci.cbs.umn.edu/software/proscan/promoterscan.htm, Prestridge, D.S. 1995, Prediction of Pol II Promoter Sequence using Transcription Factor Binding Sites. J. Mol. Biol. 249: 923-932). The deletion study can be conducted, focusing on the core regions predicted.

An expression plasmid in which a reporter gene is operably linked downstream of the thus isolated gene for the regulatory region is constructed and introduced into an appropriate cell. As used herein, the term "operably linked" means that the two elements are linked so that transcription of the reporter gene is initiated by activation of the above expression regulatory region. Any gene may be used as a reporter gene as long as it encodes a protein that allows one to observe an activation of the above regulatory region as an expression of the gene. Particularly, genes such as luciferase, β-galactosidase, GFP (Green Fluorescent Protein) are typically used as reporter genes. Mammalian cells having a deletion in the corresponding gene, for example, can be used as cells for introducing the vector.

In the established cell line obtained as above, the promoter is activated under the conditions so as to cause cell death (apoptosis), followed by the production of a signal by the reporter gene. Resulting cells are plated on 96-well multiplates to start culturing under the conditions to cause apoptosis. By adding target compounds for screening to each well, the compound that suppress or promote the production of expression product of the gene can be easily selected. As a method for selecting a compound, if GFP is used as the reporter gene, the fluorescence intensities between the presence and absence of the compound can be compared. Comparison refers to when the luminescence ration is two-folds or higher, or 1/2 or lower, preferably five-folds or higher, or 1/5 or lower, and more preferably 10-folds or higher, or 1/10 or lower. This method can be applied to not only animal cells, but also other cells, regardless of being of eukaryotic or prokaryotic origin, as long as they can express cell death in a similar system.

Candidate substances include, but are not limited to, for example, cell extracts, expression products of a gene library, synthetic low molecular weight compounds, synthetic peptides, native compounds, and so forth.

Compounds isolated by this screening are candidates for a compound that promotes or suppresses the activity or expression of a protein of the present invention (agonist or antagonist). They are also candidates as compounds that inhibit the interaction between the proteins of the present invention and certain molecules that interact with the proteins. These compounds may be possibly applied to the treatment or prevention of diseases related to the proteins of the present invention.

Furthermore, the present invention relates to a pharmaceutical use of substances that can be obtained by the screening method of the present invention. That is, the present invention relates to a use of pharmaceutical containing a compound obtainable by the above-described screening method as a principal component in the control of cell proliferation or cell death. Alternatively, the present invention relates to a therapeutic agent which is characterized by containing these compounds as the principal component, for disorders characterized by cell proliferation or cell death. In the present invention, disorders characterized by cell proliferation or cell death mean those caused by the aberrant cell proliferation or cell death. Disorders caused by the aberrant cell proliferation may include, for example, cancers resulted from the proliferation of malignant tumor cells, etc. Alternatively, disorders caused by cell death may include the brain tissue damage caused by the cerebral ischemia. Compounds that inhibit the function or the production per se of the apoptosis-associated factor of the present invention can be used for the control of the following cell death:
(1) cell death due to ischemia;
(2) cell death in chronic viral diseases and cell death in chronic diseases due to other pathogenic microorganisms;
(3) inflammation due to the autoimmune reaction and associated cell death thereof; and,
(4) cryptogenic disorders (such as Alzheimer's disease) due to cell death and resulting degeneration.

These cell deaths are caused by, for example, the following diseases. Therefore, compounds selected by the screening method of the present invention can be also used for the treatment of the following disorders:
cerebrovascular disease dementia, multiple microcerebral infarction, cerebral thrombosis, cerebral infarction, and cerebral hemorrhage;
neurodegenerative disease (Alzheimer's disease, etc.);
cardiac infarction and myocarditis;
viral hepatitis, alcoholic hepatitis, and cirrhosis;
insulin-dependent diabetes (due to the immunoreactive cell death of the pancreatic Langerhans' islet cell);
ischemic enteritis;
cell death due to systemic or localized autoimmune disorders: systemic, generalized erythema, and dermatomyositis, rheumatoid arthritis; and
AIDS.

The proteins, nucleotides, antibodies of the present invention and the compounds isolated by the above screening mentioned are useful for regulating apoptosis. When used as pharmaceutical agents, they themselves can be used as pharmaceutical agents, or they can be formulated for use by any known pharmaceutical method. For example, the compounds can be formulated by mixing with pharmacologically acceptable carriers or vehicles, specifically, sterilized water, physiological saline, plant-oil, emulsifiers, suspending agents, and used. Methods well known to one skilled in the art may be. used to administer a pharmaceutical agent to patients, for example as intra-arterial, intravenous, percutaneous injections, and so on. The dosage varies according to the body-weight and age of the patient, and also the administration method, but one skilled in the art can suitably select an appropriate dosage. If the compound can be encoded by a polynucleotide, the polynucleotide can be inserted into a vector for gene therapy to perform the therapy. The dosage and method of administration vary according to the body-weight, age, and symptoms of a patient, but one skilled in the art can select them suitably. Also, all publications cited herein are incorporated by reference.

### Best Mode for Carrying out the Invention

The present invention will be explained in detail below with reference to examples, but it is not to be construed as being limited thereto.

### [Example 1] Construction of a cDNA library by the oligo-capping method

A cDNA library was prepared from the NT-2 neuron progenitor cells (purchased from Stratagene), which is a teratocarcinoma cell derived from the fatal human testis and can differentiate into neurons by the treatment with retinoic acid. First, NT-2 cells were cultured without the induction by retinoic acid according to the manual supplied by the manufacturer, and then the cultured cells were harvested. mRNA was extracted by the method described in the reference (J. Sambrook, E. F. Fritsch & T. Maniatis, Molecular Cloning Second edition, Cold Spring Harbor Laboratory Press 1989). Poly(A)⁺ RNA was further purified from the mRNA using oligo-dT cellulose.

Poly(A)⁺RNA was used to construct a cDNA library by the oligo-capping method (Maruyama M. and Sugano S. (1994) Gene 138: 171-174). Using the Oligo-cap linker (agcaucgagu cggccuuguu ggccuacugg/SEQ ID NO: 5) and the Oligo-dT primer (gcggctgaag acggcctatg tggccttttt tttttttttt tt/SEQ ID NO: 6), BAP (Bacterial Alkaline Phosphatase) treatment, TAP (Tobacco Acid Phosphatase) treatment, RNA ligation, the first strand cDNA synthesis, and removal of RNA were performed as described in the reference (Suzuki and Kanno (1996) Protein Nucleic acid and Enzyme. 41: 197-201; Suzuki Y. et al. (1997) Gene 200: 149-156). Next, 5'- and 3'-PCR primers ((agcatcgagt cggccttgtt g/SEQ ID NO: 7) and (gcggctgaag acggcctatg t/SEQ ID NO: 8) respectively) were used to perform PCR (polymerase chain reaction) to convert the first-strand cDNA into double-stranded cDNA, which was then digested with SfiI. Then, the DraIII-digested vector pME18SFL3 was used for cloning the cDNA with correct orientation, and cDNA libraries were obtained. The clones having an insert cDNA of 1 kb length or shorter were removed for the plasmid DNAs of the clones thus obtained. Then, the nucleotide sequence of the 5'- and 3'- ends of the cDNA clones was analyzed using DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, from by PE Biosystems), performing sequencing reactions according to the instructions, and analyzing with a DNA sequencer (ABI PRISM 377, PE Biosystems).

The fullness ratio of the 5'-end sequence of the cDNA clones in the libraries constructed by the oligo-capping method was determined as follows. For all the clones in which the 5'-end sequence was identical to that of any known human mRNA in the public database, the clones were judged to be "complete/full length", if they had longer 5'-end sequences than the known human mRNA, or, even if the 5'-end sequence was shorter, if they contained a translational initiation codon. A clone that did not contain a translational initiation codon was judged to be "incomplete/not full-length". The fullness ratio ((the number of complete clones)/(the number of complete clones) + (the number of incomplete clones)) of the 5'-end of the cDNA clones was determined by comparing with the known human mRNA. As a result, the fullness ratio of the 5' end sequence of the cDNA clone in this library (NT2RM1) was determined to be 69%, indicating the extreme high ratio thereof.

Next, using a protein localization-predicting program "PSORT", developed by Nakai and Kanehisa, the presence of sequences estimated to be signal peptides characteristic of the amino termini of many secretory proteins was analyzed for all the deduced amino acid sequences initiating from each ATG codon in the 5'-end sequence of clones of the library prepared by the oligo-capping method. Thus, clones estimated to comprise signal sequences (most likely to be secretory proteins or membrane proteins) were specifically selected from the clones of the library prepared by the oligo-capping method.

Furthermore the complete cDNA sequence and the predicted amino acid sequence of the clones thus selected were determined. The final nucleotide sequences were determined by combining the following three methods, and overlapping the nucleotide sequences determined by each method.
(1) Long read sequencing from both ends of the cDNA inserts using a Licor DNA sequencer (Sequence reactions were performed according to the manual of the Licor sequencer (Aloka), and DNA sequence was determined using the sequencer.)
(2) Nested sequencing by the Primer Island method which utilizes the in vitro integration reaction of AT2 transposon (Devine S.E., and Boeke J.D. (1994) Nucleic Acids Res. 22: 3765-3772) (Clones were obtained using a kit from PE Biosystems, and sequence reactions were performed using the DNA sequencing reagents from the same company, according to the manufacturer's instructions, and DNA sequence was determined using an ABI PRISM 377 sequencer).
(3) Primer walking by the dideoxy terminator method using custom synthesized DNA primers (Sequencing reactions were performed using the DNA sequencing reagents from PE Biosystems and custom synthesized DNA primers according to the manufacturer's instructions. DNA sequence was determined using an ABI PRISM 377 sequencer).

Obtained sequences were subjected to analysis by ATGpr [A. A. Salamov, T. NISHIKAWA, M. B. Swindells, Bioinformatics, 14: 384-390 (1998); http://www.hri.co.jp/atgpr/] and PSORT, and also to BLAST search of GenBank and SwissProt. PSC0004 is a "clone derived from a human cDNA library prepared by the oligo-capping method with high fullness ratio, predicted to be a full-length cDNA clone by using the programs such as ATGpr, and predicted to be a secretory protein or membrane protein having the signal sequence at the N-terminus by using PSORT". The above results are demonstrated below:
Name of clone: PSEC0004
cDNA size: 1883 bp: C-NT2RM1000558
The number of constitutive amino acid in a putative amino acid sequence: 326
The number of ATG counted from the N-terminus: 1
Maximum ATGpr1 value: 0.94
Annotation: 532/852 (62%) similarity to human death effector domain-containing testicular molecule mRNA

### [Example 2] Functional prediction by homology analyses

Some genes associated with apoptosis have been reported to share the Death Effector Domain (DED) among them (Chinnaiyan, A. M. et al., 1995, Cell 81: 505-512; Muzio, M. et al., 1996, Cell 85: 817-827; Fernandes-Alnemri, 1996, Proc. Natl. Acad. Sci. USA 93: 7464-7469). For example, similarly to FADD and Caspase-8, which play an important role in the apoptosis signaling by CD95 (Fas), DEDD has been reported to be a novel molecule comprising DED (Alexander H. Stegh et al., 1998, The EMBO Journal Vol. 17 No. 20, 5974-5986; Peter, M. E. et al., 1995, Cell Death Differ., 2, 163-171). Therefore, using the N-terminal 76 amino acid residues on DEDD (LYSLHRMFDIVGTHLTHRDVRVLSFLFLVDVIDDHERGLIRNGRDFLLALERQGRCDESNFR QVLQLLRIITRHDLL/ SEQ ID NO: 9 (DEDD: 23-99)) as query, homology analysis (Blast 2) was performed for the amino acid sequence encoded by the above-described full-length cDNA clone. As a result, the amino acid sequence encoded by NT2RM1000558 showed about 43% homology.

Similarly to DEDD, some genes associated with apoptosis have been reported to encode comprising a specific amino acid sequence (DEXD) cleaved by caspase, which is considered to be an effector protein for apoptosis. Therefore, using this sequence (DEXD) as query, homology analysis (Blast 2) was performed for the amino acid sequence encoded by the above-described full-length cDNA clone. As a result, the DEAD sequence was also detected in proximity to the C-terminus of the amino acid sequence encoded by NT2RM1000558.

Results described above have predicted that the NT2RM1000558 clone is an apoptosis-associated gene. Furthermore, comparison of sequences between NT2RM1000558 and DEDD revealed that the former comprised a sequence having a high homology to the NLS domain, which had been found in DEDD, indicating that NT2RM1000558 retained an apoptosis-associated function.

### [Example 4] Expression in various human tissue distribution

Expression of NT2RM1000558, which had been predicted to have the apoptosis-associated function, in various human tissues was examined. Using the primer 1 (TCAGTGTGGATGAGGCTGACT/ SEQ ID NO: 10 (NT2RM1000558: at positions 1013-1033)) and primer 2 (TGCACCTGTGACAGTGCAGA/ SEQ ID NO: 11 (NT2RM1000558: at positions 1399-1380)), PCR was performed (for 30 cycles) with the Multiple Tissue cDNA Panels (Clontech) (CODE K1420-1, K1426-1) as a template DNA according to the manual provided by the manufacturer. After the reaction, the mixture was electrophoresed on agarose gel, and expression levels of NT2RM1000558 in respective tissues were confirmed from the results of electrophoresis.

As a result, no tissue specificity was observed in the expression of NT2RM1000558 was confirmed in many tissues (including brain, heart, kidney, liver, lung, pancreas, placenta, bone marrow, lymph node, leukocytes, spleen, thymus and tonsil).

### [Example 5] Functional analysis of NT2RM1000558

In the following expression analysis, the pcDNA3.1 vector (Invitrogen) was used for all cDNA constructs. The vector strongly induces the expression of foreign genes in mammalian cells under the control of CMV promoter.

The NT2RM1000558 gene inserted into the restriction enzyme Dra III site of pME18SFL3 was excised by digestion with restriction enzymes EcoRI and NotI. On the other hand, pcDNA3.1 (Invitrogen), an expression vector, was similarly digested with restriction enzymes EcoRI and NotI, and ligated with the purified gene fragment described above. The resulting pcDNA3.1 vector inserted with the entire NT2RM1000558 gene was used as intact protein expression vector.

Next, a vector for expressing the active form protein was constructed as follows. First, with the NT2RM1000558 gene inserted into the restriction enzyme Dra III site of pME18SFL3 as a template, PCR was performed using the Advantage GC rich PCR kit (Clontech). The sense primer, 90-GGTTCTGAGCTTGTTCC (SEQ ID NO: 12), which is used in PCR, comprises the nucleotide sequence corresponding to that of the 5'-noncoding region of the NT2RM1000558 gene. On the other hand, the antisense primer, TCAGTCAGCCTCATCCACACTGA-1013 (SEQ ID NO: 13), comprises the nucleotide sequence encoding the amino acid sequence VSVDEAD portion of the protein, added with the nucleotide sequence corresponding to a stop codon just downstream of it. After PCR was completed, the PCR product (active form) was subcloned into the pT7Blue-2T (Novagen), which is a TA-cloning vector. The active form NT2RM1000558 gene inserted into the pT7Blue-2T was sequenced with the SP6 primer and M13 primer using the BigDye terminator Cycle sequence kit (PE Biosystems) and PE310 Genetic Analyzer (PE Biosystems), it was confirmed that the nucleotide sequence of the active form NT2RM1000558 gene had no mutations due to PCR.

Furthermore, the active form NT2RM1000558 gene inserted in pT7Blue-2T was digested at the restriction enzymes EcoRI and XhoI sites on pT7Blue-2T with these restriction enzymes, followed by excising. Then, the expression vector pcDNA3.1 was similarly digested with the restriction enzymes EcoRI and XhoI, and ligated with the purified gene fragment described above. The resulting pcDNA3.1 vector inserted with the active form NT2RM1000558 gene was used as the active form protein expression vector.

HEK293T cells were plated at 2x 10⁵ cells per well on 6-well plates, and cultured overnight. To the Lipofectamine 2000 solution (Gibco BRL) (10 µl), the Opti-MEM solution (250 µl) was added, stirred, and allowed to stand for 5 minutes at room temperature. The plasmid DNA (either the intact or active form expression vector) (4 µg) and Opti-MEM solution (250 µl) are mixed together, added to the resulting mixture described above, and allowed to stand for further 20 minutes at room temperature. By adding the resulting Lipofectamine 2000-plasmid DNA mixture to the above-described cultured cells, followed by culturing for 30 hours, the cells were introduced with either the intact form gene or active form gene.

After removing the culture medium from the cells, PBS-EDTA solution (2 ml) was added to the cells and then suspended with detached cells from the plates by pipetting. The resulting mixture was centrifuged at 400x g for 5 minutes to remove the supernatant PBS. The cells were further suspended in added PBS (1 ml), and centrifuged at 400x g for 5 minutes to remove the supernatant PBS.

Subsequently, the cells were suspended in 1% glutaraldehyde solution (100 µl) added, and fixed by allowed to stand for 30 minutes at room temperature. Then, the cells were centrifuged at 400x g for 5 minutes to remove the supernatant fixative. The cells were further suspended in PBS (1 ml) added, and centrifuged at 400x g for 5 minutes to remove the supernatant PBS.

The cells were suspended in PBS (20 µl) added, and further mixed with 1 mM HOECHST 33258 solution (SIGMA) (fluorescence dye) (5 µl). With a drop of the cell suspension overlaid with a cover glass on a slide glass, the nuclear form (nuclear fragmentation) was observed under a fluorescence microscope.

Nuclear fragmentation due to apoptosis was not observed in the cells into which the full-length sequence of NT2RM1000558 gene (encoding 326 amino acid residues) had been introduced by the above method, but was observed in the cells into which an activated form NT2RM1000558 gene (encoding 303 amino acid residues), which lacked the region downstream from the DEAD portion in the NT2RM1000558 gene, had been introduced. As a control, nuclear fragmentation due to apoptosis was also observed in cells into which the Caspase-3 gene had been introduced.

Considering the substrate specificity of the caspase family to the cleavage recognition sequence, it is assumed that the DEAD sequence, the cleavage sequence recognized by the activated caspase in the NT2RM1000558 gene is a molecule, which is activated by the cleavage with Caspase-3, Caspase-6, and Caspase-7, playing the ultimate role in the apoptosis execution similarly to the poly (ADP-ribose) polymerase (PARP) and DNA fragmentation factor.

A protein synthesized from the NT2RM1000558 gene via *in vitro* transcription/translation system indicated a 36 kd band in SDS-PAGE. Thus, it was confirmed that, in this gene, the actual protein translation initiates from the initiator methionine in the reading frame as predicted.

### Industrial Applicability

The present invention has provided a novel apoptosis-associated factor, which comprises the recognition site for the group 2 caspase constituting downstream of the apoptosis execution cascade. Therefore, the apoptosis-associated factor of the present invention may be an important protein involved in the final stage of apoptosis. Actually, symptoms of typical apoptosis have become to be observed in cells, in which active form molecules of the apoptosis-associated factor of the present invention have been overexpressed. Therefore, compounds that control the action of the apoptosis-associated factor of the present invention can be screened using apoptosis as an indicator. Compounds that may be obtained by the screening method of the present invention are useful as drugs to control the cell proliferation or cell death.

For example, compounds that suppress the action of the apoptosis-associated factor of the present invention can be used as drugs to prevent the progression of apoptosis. Since the apoptosis-associated factor of the present invention functions at the final stage of apoptosis execution cascade, compounds that interfere with the action may effectively suppress apoptosis. The apoptosis-associated factor of the present invention is widely distributed over respective human tissues, and is predicted to play a critical role for a variety of the above-described disorders with impairments spread by apoptosis. The apoptosis-associated factor of the present invention was isolated from neurocytes in particular. The control of apoptosis induced with cerebral ischemia is important objects in the prevention and treatment of injuries of brain tissues. The apoptosis-associated factor of the present invention is important as a target molecule for the prevention and treatment of damages of brain tissues.

On the contrary, compounds that enhance the action of apoptosis-associated factor of the present invention or stimulate the production thereof are useful as drugs to promote cell death. The apoptosis-associated factor of the present invention strongly induces apoptosis. Therefore, the apoptosis-associated factor per se of the present invention or compounds promoting the production thereof can be used as drugs to suppress the cell proliferation, for example, in cell proliferative disorders such as cancer.

Although apoptosis can be induced by a variety of causes, drugs which act at the final stage of the execution cascade thereof may control apoptosis regardless of its causes. Therefore, compounds capable of controlling actions of the apoptosis-associated factor of the present invention can be expected to achieve the control of apoptosis in a broad range of disorders.

## Claims

1. A polynucleotide encoding a protein having apoptosis-inducing activity, said polynucleotide selected from the group consisting of the following (a) to (d):
(a) a polynucleotide comprising the nucleotide sequence of SEQ ID NO: 1;
(b) a polynucleotide encoding a protein comprising the amino acid sequence of SEQ ID NO: 2;
(c) a polynucleotide encoding a protein comprising the amino acid sequence of SEQ ID NO: 2 in which one or more amino acids are substituted, deleted, inserted, and/or added; and
(d) a polynucleotide hybridizing under stringent conditions with a polynucleotide comprising the nucleotide sequence of SEQ ID NO: 1.

2. The polynucleotide of claim 1, said polynucleotide having 60% or more homology to the nucleotide sequence of SEQ ID NO: 1.

3. A polynucleotide encoding a precursor of a protein having apoptosis-inducing activity, said polynucleotide selected from the group consisting of the following (e) to (h):
(e) a polynucleotide comprising a protein coding region of the nucleotide sequence of SEQ ID NO: 3;
(f) a polynucleotide encoding a protein comprising the amino acid sequence of SEQ ID NO: 4;
(g) a polynucleotide encoding a protein comprising the amino acid sequence of SEQ ID NO: 4 in which one or more amino acids are substituted, deleted, inserted, and/or added; and
(h) a polynucleotide hybridizing under stringent conditions with a polypeptide comprising the nucleotide sequence of SEQ ID NO: 3.

4. The polynucleotide of claim 3, said polynucleotide having 60% or more homology to the nucleotide sequence of SEQ ID NO: 3.

5. A polynucleotide encoding a partial peptide of a protein encoded by the polynucleotide of claims 1 or 3.

6. Apolynucleotide encoding molecular-evolutionarily the same gene as a gene comprising the nucleotide sequence of SEQ ID NO: 3.

7. A polynucleotide encoding a protein that comprises the amino acid sequence of SEQ ID NO: 4 in which one or more amino acids are substituted, deleted, inserted, and/or added and that has a character dominant negative to that of a protein comprising the amino acid sequence of SEQ ID NO: 4.

8. The polynucleotide of claim 7, said polynucleotide encoding the amino acid sequence of SEQ ID NO: 4 in which the amino acid sequence at position 300 to 303 has been modified to a caspase-resistant amino acid sequence.

9. A protein encoded by the polynucleotide according to any one of claim 1, claim 3, claim 5, claim 6, and claim 7.

10. A vector comprising the polynucleotide according to any one of claim 1, claim 3, claim 5, claim 6, and claim 7.

11. A transformant retaining the polynucleotide according to any one of claim 1, claim 3, claim 5, claim 6, and claim 7, or the vector of claim 10.

12. A method for producing the protein of claim 9, said method comprising the steps of:
(1) culturing the transformant of claim 11; and
(2) recovering an expression product.

13. A polynucleotide comprising a polynucleotide complementary to the polynucleotide according to any one of claim 1, claim 3, claim 5, claim 6, and claim 7 or a complementary strand thereof, said polynucleotide being at least 15 bases long.

14. An antibody against the protein of claim 9.

15. An immunological assay method comprising a step of observing an immunological reaction between the protein of claim 9 and the antibody of claim 14.

16. A method for screening a substance regulating apoptosis, said method comprising the following steps of:
(1) contacting, with a candidate substance, cells expressing a protein encoded by the polynucleotide of claim 1 or claim 3; and
(2) culturing said cells under conditions inducing apoptosis to select a candidate substance that suppresses or promotes apoptosis.

17. A method for screening a substance regulating apoptosis, said method comprising the following steps of:
(1) contacting, with a candidate substance, cells into which a vector comprising an expression control region for a gene comprising the nucleotide sequence of SEQ ID NO: 3 and a reporter gene operably linked downstream thereof has been introduced;
(2) measuring activity of said reporter gene; and
(3) selecting a candidate substance that increases or decreases the reporter gene activity in the step (2) compared with a control.

18. Use of a compound that can be obtained by the method of claim 16 or claim 17, in regulation of cell proliferation or cell death.

19. A therapeutic agent for disorders **characterized by** cell proliferation or cell death, said agent comprising, as a principal ingredient, a compound that can be obtained by the method of claim 16 or claim 17.
